# EUROPEAN PATENT SPECIFICATION

(11) **EP 1 059 901 B1**
(45) Date of publication and mention of the grant of the patent: **21.07.2004**
(21) Application number: 99909813.0
(22) Date of filing: 03.03.1999
(51) Int. Cl.: A61F 5/00, A61F 5/41

(54) **DEVICE FOR TREATMENT OF ERECTILE DYSFUNCTION**
VORRICHTUNG ZUR BEHANDLUNG VON EREKTIONSTÖRUNGEN
APPAREIL DE TRAITEMENT DE LA DYSERECTION

(30) Priority: 03.03.1998 ZA 9801789
(43) Date of publication of application: 20.12.2000
(73) Proprietor: Bonthuys, Barend Willem, Moreleta Park, 0044 (ZA)
(72) Inventor: Bonthuys, Barend Willem, Moreleta Park, 0044 (ZA)
(74) Representative: Haley, Stephen
(86) International application number: PCT/US1999/004736
(87) International publication number: WO 1999/044550

(56) References cited:
- WO-A-97/25007
- CH-A- 347 300
- DE-A- 4 344 686
- US-A- 4 753 227
- US-A- 5 095 895
- US-A- 5 624 378
- US-A- 5 782 621
- US-A- 5 836 864

## Description

This invention relates to a device for the treatment of erectile dysfunction or for penile exercise in the human male.

A problem that occasionally occurs in human males is the inability to attain an erection of the penis.

It is thus an object of the invention to provide a device which may be used particularly but not necessarily exclusively in the treatment of erectile dysfunction and for penile exercise, and which is not unwieldy or cumbersome when used in intimate situations.

US-A-5095895, US-A-4753227 and US-A-5624378 all disclose devices for treating erectile dysfunctions.

According to one aspect of the invention thee is provided a device (10,100) for the treatment of erectile dysfunction or for penile exercise, the device comprising: a vessel (12) having an outer surface which is substantially cylindrical for at least part of its length, the vessel (12) defining a vacuum chamber, and having a mouth opening (12,4) leading into the vacuum chamber (14), the vacuum chamber (14) being shaped and dimensioned to accommodate a human penis in an erect state; and pumping means (50); characterised in that the pumping means comprises an annular pumping member (16) mounted on and extending concentrically around the cylindrical portion of the outer surface of the vessel (12) intermediate the ends thereof so that an annular suction chamber (20) is defined between the outer surface of the vessel (12) and the pumping member (16), the pumping member (16) and vessel (12) being slidingly displaceable relative to one another which relative displacement changes the volume of the suction chamber (20); at least one pumping passage (28) extending from the vacuum chamber (14), at a position intermediate the ends thereof, to the suction chamber (20); at least one exhaust passage (29) extending from the suction chamber (20); first one way valve means (32) which permits fluid flow from the vacuum chamber (14) to the suction chamber (20) *via* the, or each, pumping passage (28); and second one way valve mens which permits fluid to be discharged from the suction chamber (20) via the or each exhaust passage (29) to atmosphere.

The device may include a seal means positioned in the vicinity of the mouth opening and configured sealingly to engage with an outer surface of a human penis inserted in the vacuum chamber.

As the vacuum drawn in the vacuum chamber increases it becomes more difficult to draw air out of the vacuum and the effort required to displace the pumping member in the direction required to increase the volume of the suction chamber increases. By arranging the pumping means such that the volume of the pumping chamber increases when the pumping member is displaced towards the mouth of the vessel the primary loads applied to the device, in use, are towards a user's body which helps to minimize any discomfort the user may experience.

The pumping vessel may have a cylindrical wall and the or each pumping passage may extend through the wall and have a mouth opening into the suction chamber, the first one way valve means being in the form of at least one elastically deformable band extending around the cylindrical wall to cover the or each mouth.

The second one way valve means may include an O-ring configured to provide an air tight seal between the pumping member and the vessel when being displaced relative to one another to increase the volume of the pumping chamber and to permit air to be discharged through the at least one exhaust passage when the pumping member and the vessel are being displaced relative to one another to decrease the volume of the pumping chamber.

In order to ensure the safe operation of the device it is desirable that the pressure in the vacuum chamber not decrease below a predetermined minimum pressure, typically of the order of -0.57 bar. However, the Inventor believes that a vacuum of -0.4 bar will work satisfactorily.

Accordingly, the device may include vacuum restriction means for restricting the vacuum which can be drawn in the vacuum chamber.

The vacuum restriction means may include a stroke limiting member positioned in the suction chamber to restrict the stroke through which the pumping member is displaceable relative to the vessel.

The seal means may include a sealing constriction ring which is readily dismountably mounted on the vessel in the vicinity of the mouth opening, the constriction ring including a generally ring-shaped body having a circumferentially extending engagement formation for releasable sealing engagement with the vessel in the vicinity of the mouth opening and a constriction ring opening configured sealingly to abut against the outer surface of a human penis.

The body of the constriction ring may be formed of a unitary moulding of an elastomeric material. In one embodiment of the invention the elastomeric material may be a silicone rubber and have a hardness of 50 A Shore.

The size of the constriction ring opening will depend on the intended application. Hence, where intended for use in the treatment of erectile dysfunction the opening will be sufficiently small to inhibit blood flow from the penis once an erection is attained in order to maintain the erection. In contrast, when the device is intended to be used for penile exercise the opening will be sufficiently small to seal against a surface of the penis and sufficiently large to permit blood to drain from the erect penis.

The constriction ring opening may have a diameter of between 13 and 24 mm.

The device may include a plurality of interchangeable sealing constriction rings each having a constriction ring opening of a different size.

The invention will now be described, by way of nonlimiting example, with reference to the accompanying diagrammatic drawings.

In the drawings,
Figure 1 shows a three-dimensional view of a device for the treatment of erectile dysfunction or for penile exercise in accordance with the invention;
Figure 2 shows a side view of the device of Figure 1;
Figure 3 shows a sectional side view of part of the device of Figure 1;
Figure 4 shows, on an enlarged scale, a sectional side view of part of a sealing constriction ring forming part of the device of Figure 1; and
Figure 5 shows a sectional side view of another device in accordance with the invention.

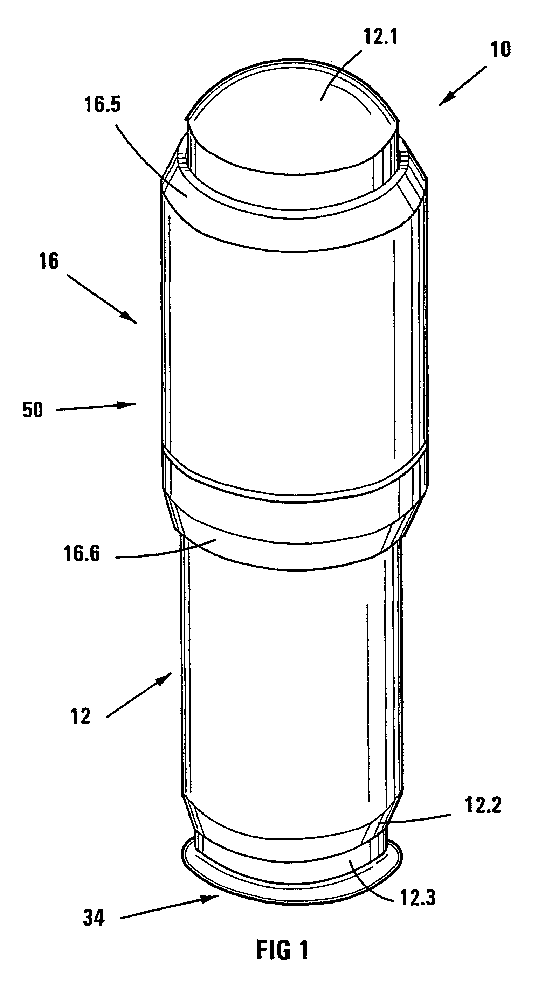

Referring to Figures 1 to 4 of the drawings, reference numeral 10 generally indicates a device in accordance with the invention for use in the treatment of erectile dysfunction or for penile exercise.

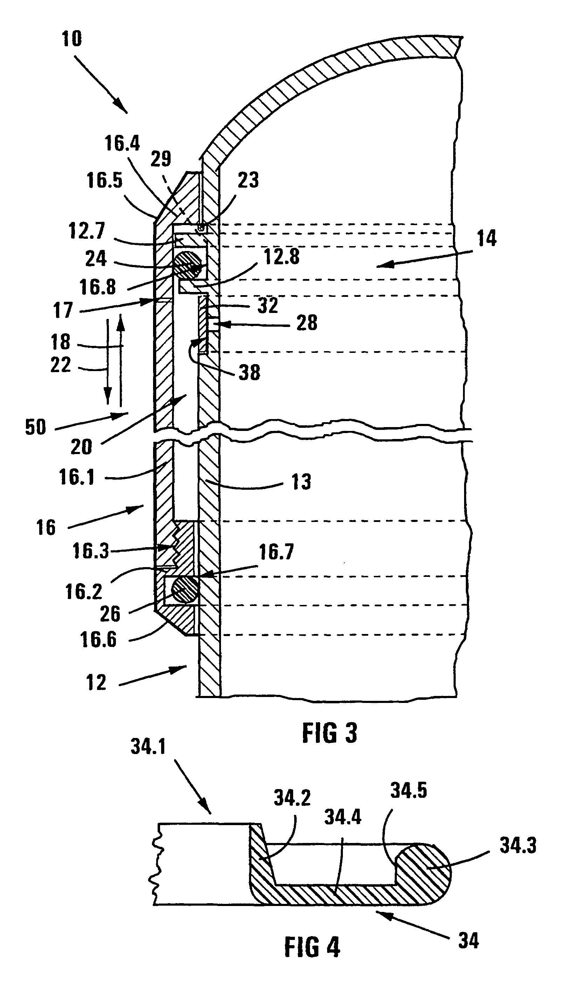

The device 10 includes a hollow generally cigar shaped vessel 12 defining an elongate primary or vacuum chamber 14 (Figure 3). The device 10 further includes pumping means, generally indicated by reference numeral 50 for pumping air out of the vacuum chamber 14.

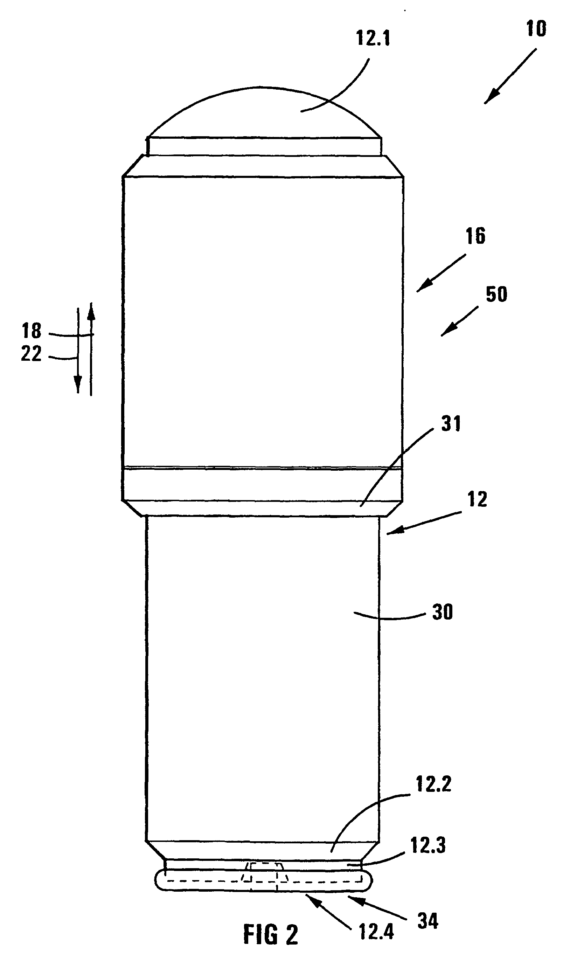

The vessel 12 has a generally cylindrical wall 13. The pumping means 50 includes a cylindrical pumping member or sleeve 16 which is mounted on and slidingly displaceable relative to the vessel 12 in the directions of the arrows 18, 22 (Figures 2 and 3). Referring, in particular, to Figure 3 the vessel 12 and the sleeve 16 are configured so that, together, they form an annular secondary or suction chamber 20. As is also evident from Figure 3, displacement of the pumping member in the direction of the arrow 22 will increase the volume of the suction chamber 20 and displacement in the direction of the arrow 18 will decrease the volume of the suction chamber 20. The pumping means 50 further includes sealing means in the form of a pair of O-rings 24, 26, described in further detail below, and pumping passages 28 in the wall 13 of the vessel 12 which provide a flow path for air to flow from the vacuum chamber 14 to the suction chamber 20. Four pumping passages 28 are provided, arranged in diametrically opposed pairs. the pumping means 50 includes upper exhaust passages, described in further detail below, indicated by the dotted lines 29 in Figure 3 which allow air in the suction chamber 20 to be discharged to atmosphere when the pumping member 16 is moved in the direction of the arrow 18. The pumping means 50 further includes one way valve means in the form of a flat rubber band 32, described in further detail below, extending around the cylindrical vessel 12 to cover the pumping passages 28.

The vessel 12 has an open bottom end or mouth opening 12.4 (Figure 2) which leads into an end of the vacuum chamber 14. Seal means in the form of a sealing constriction ring 34 is mounted on the vessel 12 in the vicinity of the mouth opening 12.4, as described in further detail below.

As can be seen, in particular, in Figure 2, the cylindrical vessel 12 has a closed dome-shaped upper end 12.1 and a lower shoulder 12.2 adjacent the opening 12.4 with a narrower cylindrical part 12.3 extending from it and defining the opening 12.4.

As can be seen in Figure 3, the sleeve 16 comprises a top part 16.1 and a bottom part 16.2 which are screw-threadedly joined by complementary screw threads generally indicated by reference numeral 16.3 with a lower portion of the upper part 16.1 overlapping an upper portion of the lower part 16.2 at the screw threads 16.3. This allows the device 10 to be disassembled. The upper part 16.1 has an upper inwardly directed collar 16.4 with a tapered upper face 16.5. The lower part 16.2 has a complementary tapered lower face 16.6. The lower part 16.2 has a circumferentially extending inwardly directed recess 16.7 and the O-ring 26 is positioned in the recess 16.7. The O-ring 26 is configured so that it sealingly and slidingly abuts the wall 13 of the cylindrical vessel 12 to provide an airtight seal between the vessel 12 and the pumping member or sleeve 16.

A collar 12.7 projects outwardly from the wall 13 below the inwardly directed collar 16.4 of the sleeve 16. The exhaust passages 29 are formed by spaced recesses in the collar 12.7. A second outwardly projecting collar 12.8 is located below the collar 12.7 and the wall 13 of the vessel 12 between the collars 12.7 and 12.8 is recessed. The collar 12.7 projects slightly further from the wall 13 of the vessel 12 than does the collar 12.8 as can be seen, in particular, in Figure 3. This allows the sleeve 16 to slide smoothly over the collar 12.7 which thus acts as a guide for accurate "centering" of the sleeve 16. The collars 12.7, 12.8 define between them an annular cavity 16.8 and the O-ring 24 is positioned in the cavity 12.8. The O-ring 24 has a diameter which is selected so that it abuts slidingly and sealingly against the inside face of the sleeve 16. A further recessed portion 38 in the wall 13 of the vessel 12 is provided below the collar 12.8 and the pumping passages 28 extend through the wall 13 of the vessel 12 in the recessed portion 38. The rubber band 32 is located in the recessed portion 38 and covers the pumping passages 28. A vacuum relief hole 17 is provided in the top part 16.1 of the sleeve 16 adjacent the upper O-ring 24 to release vacuum when the sleeve 16 is in its lowest position.

A circumferentially extending rubber stop member 23 is provided above the collar 12.7 to dampen the sound of the sleeve when it is reciprocated.

Thus, in use, if the sleeve 16 is moved upwardly i.e. in the direction of the arrow 18, the O-ring 24 is displaced upwardly so that it abuts against the collar 12.7 and is clear of the collar 12.8. In addition, the volume of the exhaust chamber 20 is reduced and air in the exhaust chamber is driven past the O-ring 24 and the collar 12.8 and out through the exhaust passages 29. When the sleeve 16 is moved in the direction of the arrow 22, the O-ring 24 is displaced downwardly and abuts sealingly against the collar 12.8 thereby forming an airtight seal between the sleeve 16 and the vessel 12. In addition, the volume of the exhaust chamber 20 is increased so that air is sucked via the pumping passages 28, under and past the rubber band 32, which acts as a one way valve by allowing air flow from the vacuum chamber 14 to the suction chamber 20 but preventing air flow in the opposite direction. On the return stroke of the sleeve 16 air is again exhausted via the exhaust openings 29. The O-ring 24 hence functions as a one way valve. In this way by reciprocating movement of the sleeve 16, air is drawn out of the vacuum chamber 14 thereby, when the mouth is closed, incrementally reducing the pressure in the vacuum chamber 14. It will be appreciated that if desired the O-ring 26 can function as a one way valve in addition to the O-ring 24.

Referring now to Figure 4 the constriction ring 34 comprises a body of rubber, e.g. silicone rubber. The ring 34 includes a central opening 34.1 surrounded by a tubular central portion or collar 34.2, a circumferentially extending upwardly directed ring-shaped formation or skirt 34.3 and a flat annular connecting web or recessed portion 34.4 between the collar 34.2 and the ring-shaped formation 34.3. The ring-shaped formation has an inner surface or wall 34.5. The outside diameter of the constriction ring 34 is about 58 mm. The diameter of the wall 34.5 is about 47 mm. This diameter is slightly smaller than the diameter of the cylindrical part 12.3 of the vessel 12 so that the constriction ring 34 is sealingly received or engaged on the end of the cylindrical part 12.3 of the vessel 12 by a friction fit. In different embodiments of the invention, the opening 34.1 has a diameter of 13 to 24 mm.

In use, the flaccid penis of a user (not shown) is drawn into the vessel 12 of the device 10 through the opening 34.1 of the constriction ring 34 by operating the sleeve 16 to reduce the air pressure in the pumping chamber 14 the penis is drawn into the vessel 12 and by virtue of the reduced pressure in the vacuum chamber 14 blood is drawn into the penis to achieve an erection of the penis in the vacuum chamber 14. The constriction ring 34 seats sealingly around the penis. The pumping action causes a pressure drop in the vacuum chamber 14 to about -0.4 bar. This is sufficient to draw the penis into the vessel 12 and to draw blood into the penis to cause an erection.

The skirt 34.2 is deformed by the drawing of the penis into the opening 34.1 thereby causing the seal-penis contact area to be increased by the draping of the skirt 34.2 onto the penis, collar-fashion. Once an erection is achieved, the vessel 12 is simply disengaged from the constriction ring 34 which remains in place on the erect penis. In this case the diameter of the opening 34.1 is selected by a user of the device 10 so that blood flow from the penis is constricted. In the case of penile exercise, the diameter of the opening 34.1 is selected by a user so that the constriction ring engages sealingly with the penis during the pumping action so that an erection is achieved but not so that blood flow from the penis is constricted when the vacuum is relieved. Thus when the vacuum is relieved blood flow from the penis relaxes the erection. Such penile exercise is believed to function as a treatment for erectile dysfunction.

In an embodiment of the invention (not shown), the vessel 12 is provided with a removable vacuum gauge for measuring the vacuum inside the vessel 12. Once a user is familiar with the use of the device, the vacuum gauge may be removed.

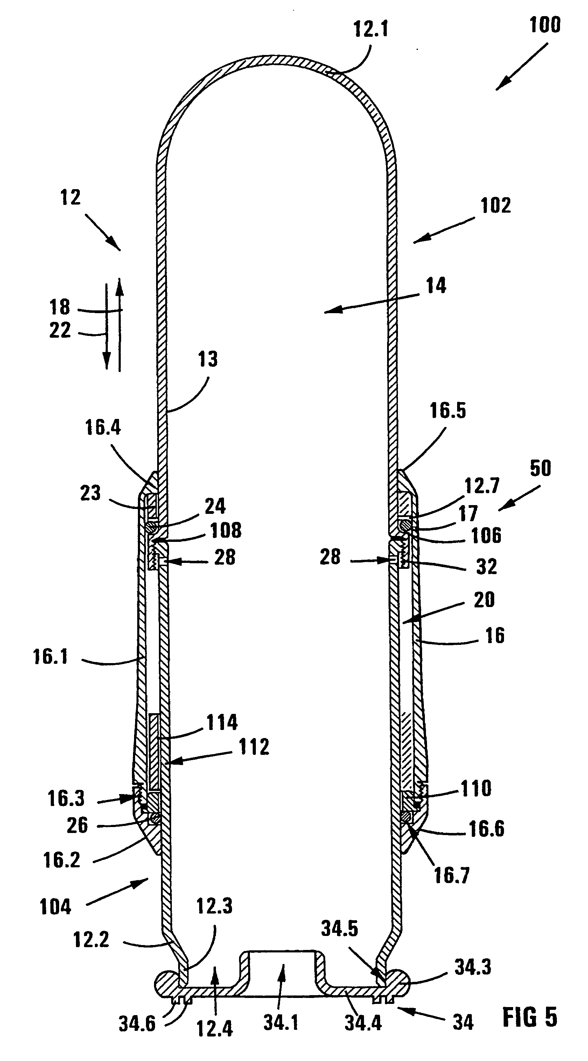

Reference is now made to Figure 5 of the drawings, in which reference numeral 100 refers generally to another device in accordance with the invention for use in the treatment of erectile dysfunction or for penile exercise. Unless otherwise indicated, the same reference numerals used above are used to designate similar parts.

In this embodiment of the invention, the vessel 12 is formed in two parts 102 and 104 which are screw-threadedly connected together. The part 102 defines an annular shoulder 106, the O-ring 24 being held captive between the shoulder 106 and the collar 12.7. A seal 108 is provided between the two parts 102, 104.

The shoulder 106 functions in the same manner as the collar 12.8 of Figures 1 to 3 such that the O-ring 24 cooperates with the shoulder 106 to form a non-return or one way valve means. The O-ring 26 is held captive between the lower part 16.2 and an annular insert 110 which is sandwiched between the upper part 16.1 and the lower part 16.2 when they are screwed together. If desired, the lower part 16.2 and insert 110 can be configured such that the O-ring 26 also functions as a non-return valve.

This arrangement, the Inventor believes, will facilitate manufacture of the device 100.

In addition, the device 100 includes vacuum restriction means, generally indicated by reference numeral 112 for restricting the vacuum which can be drawn in the vacuum chamber 14. It will be appreciated that, in use, air will be drawn from the vacuum chamber 14 into the suction chamber 20 only when the pressure in the suction chamber 24 is below that in the vacuum chamber 14. Accordingly, by limiting the maximum vacuum which can be drawn in the suction chamber 20 the maximum vacuum which can be drawn in the vacuum chamber 14 is also limited. In order to achieve this, the vacuum restriction means 112 includes a stoke limiting member 114 which is positioned in the suction chamber 20 and is designed to limit the maximum stroke of the pumping member or sleeve 16 relative to the vessel 12. It is hence a relatively simple matter to calculate the stroke volume of the sleeve 16 and hence the maximum vacuum which can be drawn.

A further difference between the device 100 and the device 10 is that, in the case of the device 100 the pumping member or sleeve 16 is moved closer towards the mouth 12.4 and is arranged that at the extremities of its stroke it does not protrude beyond the ends of the vessel 12. This reduces the risk that an article of clothing or a body part may be caught between the sleeve 16 and the vessel 12.

In this embodiment of the invention, even though the sleeve 16 is moved closer to the mouth 12.4, at the limit of its pumping stroke, i.e. when closest to the mouth 12.4, the sleeve 16 is spaced from the mouth end of the vessel as shown in Figure 5. This reduces the risk that the sleeve 16 will strike the testes or another part of the body of a user.

In addition, the diameter of the outer surface of the sleeve 16 increases towards the mouth 12.4. As mentioned above, the maximum effort applied to the sleeve 16 is when it is being displaced in the direction of arrow 22. Accordingly this gentle taper of the sleeve 16 reduces the risk that a user's hand will slip on the sleeve 16.

A further difference between the device 100 and the device 10 is that, in the case of the device 100, the sealing constriction ring 34 is provided with a pair of annular ribs or gripping formations 34.6 which assist in removing the ring 34 on the vessel 12.

The device 100 will be used in substantially the identical fashion to the device 10.

The Applicant believes that it is an advantage of the invention as illustrated that the device due to its size, form and simplicity of use can be easily used in an intimate situation. It is a particular advantage that the pumping vessel is easily detached from the constriction ring leaving the constriction ring in place.

The Applicant believes that it is a further advantage of the invention as illustrated that the sealing ring may be removably attached to the device to form a unit for ease of use.

## Claims

1. A device (10,100) for the treatment of erectile dysfunction or for penile exercise, the device comprising:
a vessel (12) having an outer surface which is substantially cylindrical for at least part of its length, the vessel (12) defining a vacuum chamber, and having a mouth opening (12,4) leading into the vacuum chamber (14), the vacuum chamber (14) being shaped and dimensioned to accommodate a human penis in an erect state; and
pumping means (50); **characterised in that** the pumping means comprises
an annular pumping member (16) mounted on and extending concentrically around the cylindrical portion of the outer surface of the vessel (12) intermediate the ends thereof so that an annular suction chamber (20) is defined between the outer surface of the vessel (12) and the pumping member (16), the pumping member (16) and vessel (12) being slidingly displaceable relative to one another which relative displacement changes the volume of the suction chamber (20);
at least one pumping passage (28) extending from the vacuum chamber (14), at a position intermediate the ends thereof, to the suction chamber (20);
at least one exhaust passage (29) extending from the suction chamber (20);
first one way valve means (32) which permits fluid flow from the vacuum chamber (14) to the suction chamber (20) via the, or each, pumping passage (28); and
second one way valve mens which permits fluid to be discharged from the suction chamber (20) via the or each exhaust passage (29) to atmosphere.

2. A device (10,100) according to claim 1, wherein the or each pumping passage (28) is positioned and the pumping member (16) is arranged so that displacement of the pumping member (16) towards the mouth (12,4) of the vessel (12) increases the volume of the suction chamber (20) and causes air to flow from the vacuum chamber (14) through the or each pumping passage (28) into the suction chamber (20) and displacement of the pumping member (16) away from the mouth (12,4) of the vessel (12) decreases the volume of the suction chamber (20) and causes air to be discharged from the suction chamber (20) through the exhaust passage (29) to atmosphere.

3. A device according to claim 1 or claim 2, wherein the vessel (12) has a cylindrical wall (13) and the or each pumping passage (28) extends through the wall (13) and has a mouth opening into the suction chamber (20), the first one way valve means being in the form of at least one elastically deformable band (32) extending around the cylindrical wall (13) to cover the or each mouth.

4. A device according to any of the preceding claims, wherein the second one way valve means includes an O-ring (24) configured to provide an air tight seal between the pumping member (16) and the vessel (12) when being displaced relative to one another to increase the volume of the suction chamber (20) and to permit air to be discharged through the at least one exhaust passage (29) when the pumping member (16) and the vessel (12) are being displaced relative to one another to decrease the volume of the suction chamber (20).

5. A device according to any of the preceding claims, wherein it includes vacuum restriction means (112) for restricting the vacuum which can be drawn in the vacuum chamber (14).

6. A device according to claim 5, wherein the vacuum restriction means includes a stroke limiting member (114) positioned in the suction chamber (20) to restrict the stroke through which the pumping member 16) is displaceable relative to the vessel (12).

7. A device according to any of the preceding claims, wherein at the extremities of its stroke, the pumping member (16) does not protrude beyond the ends of the vessel (12).

8. A device according to any of the preceding claims, wherein it includes seal means positioned in the vicinity of the mouth opening (12,4) and configured sealingly to engage with an outer surface of a human penis inserted into the vacuum chamber (14).

9. A device according to claim 8, wherein the seal means includes a sealing constriction ring (34) which is dismountably mounted on the vessel (12) in the vicinity of the mouth opening (12,4), the constriction ring (34) including a generally ring-shaped body having a circumferentially extending engagement formation (34.3) for releasable sealing engagement with a vessel (12) in the vicinity of the mouth opening (12,4) and a constriction ring opening (34.1) configured sealingly to abut against the outer surface of a human penis.

10. A device according to claim 9, wherein the body is formed of a unitary moulding of an elastomeric material.

11. A device according to claim 9 or claim 10, wherein the constriction ring opening (34,1) has a diameter of between 12 and 24 mm.

## Revendications

1. Dispositif (10, 100) pour le traitement du dysfonctionnement érectile ou pour la pratique pénienne, comprenant :
- un récipient (12) possédant une surface externe qui est sensiblement cylindrique sur au moins une partie de sa longueur, le récipient (12) définissant une chambre à dépression et possédant une embouchure (12.4) conduisant à la chambre à dépression (14), la chambre à dépression (14) étant conformée et dimensionnée pour loger un pénis humain dans un état d'érection ; et
- un moyen de pompage (50) ;
**caractérisé en ce que** le moyen de pompage comprend :
- une pièce annulaire de pompage (16) montée sur et s'étendant de façon concentrique autour de la partie cylindrique de la surface externe du récipient (12) entre ses extrémités de telle façon qu'une chambre annulaire d'aspiration (20) soit définie entre la surface externe du récipient (12) et la pièce de pompage (16), la pièce de pompage (16) et le récipient (12) pouvant se déplacer de façon coulissante l'un par rapport à l'autre, ce déplacement relatif modifiant le volume de la chambre d'aspiration (20) ;
- au moins un passage de pompage (28) s'étendant de la chambre à dépression (14) sur une position entre ses extrémités vers la chambre d'aspiration (20) ;
- au moins un passage de sortie (29) s'étendant de la chambre d'aspiration (20) ;
- un premier moyen de clapet anti-retour (32) autorisant un passage du fluide de la chambre à dépression (14) vers la chambre d'aspiration (20) par l'intermédiaire du ou de chaque passage de pompage (28) ; et
- un second moyen de clapet anti-retour permettant une décharge du fluide de la chambre d'aspiration (20) par l'intermédiaire du ou de chaque passage de sortie (29) vers l'atmosphère.

2. Dispositif (10, 100) selon la revendication 1, dans lequel le ou chaque passage de pompage (28) est positionné et la pièce de pompage (16) est disposée de telle façon que la course de la pièce de pompage (16) vers l'embouchure (12.4) du récipient (12) augmente le volume de la chambre d'aspiration (20) et provoque un passage d'air de la chambre à dépression (14) par l'intermédiaire du ou de chaque passage de pompage (28) dans la chambre d'aspiration (20) et une course de la pièce de pompage (16) à l'écart de l'embouchure (12.4) du récipient (12) réduit le volume de la chambre d'aspiration (20) et provoque un drainage de l'air de la chambre d'aspiration (20) par l'intermédiaire du passage de sortie (29) dans l'atmosphère.

3. Dispositif selon la revendication 1 ou 2, dans lequel le récipient (12) possède une paroi cylindrique (13) et le ou chaque passage de pompage (28) s'étend à travers la paroi (13) et possède une embouchure dans la chambre d'aspiration (20), le premier moyen de clapet anti-retour prenant la forme d'au moins une bande déformable de façon élastique (32) s'étendant autour de la paroi cylindrique (13) afin de couvrir la ou chaque embouchure.

4. Dispositif selon l'une quelconque des revendications précédentes, dans lequel le second moyen de clapet anti-retour comprend un joint annulaire (24) configuré pour constituer un joint étanche à l'air entre la pièce de pompage (16) et le récipient (12) lors d'un déplacement relatif l'un par rapport à l'autre afin d'augmenter le volume de la chambre d'aspiration (20) et de permettre un drainage de l'air à travers au moins le passage de sortie (29) lorsque la pièce de pompage (16) et le récipient (12) sont déplacés l'un par rapport à l'autre afin de réduire le volume de la chambre d'aspiration (20).

5. Dispositif selon l'une quelconque des revendications précédentes, comprenant un moyen de restriction de dépression (112) pour restreindre la dépression pouvant être créée dans la chambre à dépression (14).

6. Dispositif selon la revendication 5, dans lequel le moyen de restriction de dépression comprend une pièce de limitation de course (114) placée dans la chambre d'aspiration (20) afin de restreindre la course selon laquelle la pièce de pompage (16) peut se déplacer par rapport au récipient (12).

7. Dispositif selon l'une quelconque des revendications précédentes, dans lequel, aux extrémités de sa course, la pièce de pompage (16) ne dépasse pas au-delà des extrémités du récipient (12).

8. Dispositif selon l'une quelconque des revendications précédentes, comprenant un moyen d'étanchéité positionné au voisinage de l'embouchure (12.4) et configuré, de façon étanche, pour coopérer avec la surface externe d'un pénis humain inséré dans la chambre à dépression (14).

9. Dispositif selon la revendication 8, dans lequel le moyen d'étanchéité comprend un anneau d'étranglement d'étanchéité (34) monté, de façon amovible, sur le récipient (12) au voisinage de l'embouchure (12.4), l'anneau d'étranglement (34) comprenant un corps de forme globalement annulaire possédant une formation d'engagement s'étendant selon la circonférence (34.3) pour engager, de façon étanche et amovible, un récipient (12) au voisinage de l'embouchure (12.4) et une ouverture d'anneau d'étranglement (34.1) configurée, de façon étanche, pour butter contre la surface externe d'un pénis humain.

10. Dispositif selon la revendication 9, dans lequel le corps est formé d'un moulage unitaire en un matériau d'élastomère.

11. Dispositif selon la revendication 9 ou 10, dans lequel l'ouverture d'anneau d'étranglement (34.1) possède un diamètre compris entre 12 et 24 mm.

## Patentansprüche

1. Eine Vorrichtung (10, 100) zur Behandlung von Erektionsstörungen oder für penile Übung, die Vorrichtung umfasst:
einen Behälter (12) mit einer Außenfläche, die für wenigstens einen Teil ihrer Länge im Wesentlichen zylindrisch ist, wobei der Behälter (12) eine Vakuumkammer begrenzt und ein Mundstück (12.4) aufweist, das in die Vakuumkammer führt, wobei die Vakuumkammer (14) geformt und bemessen ist, um einen menschlichen Penis in erigiertem Zustand aufzunehmen, und
die Pumpeinrichtung (50), **dadurch gekennzeichnet, dass** die Pumpeinrichtung Folgendes umfasst:
ein ringförmiges Pumpelement (16), befestigt auf dem zylindrischen Abschnitt der Außenfläche des Behälters (12) und sich konzentrisch um denselben herum zwischen dessen Enden erstreckend, so dass eine ringförmige Saugkammer (20) zwischen der Außenfläche des Behälters (12) und dem Pumpelement (16) begrenzt wird und das Pumpelement (16) und der Behälter (12) relativ zueinander gleitend verstellbar sind, wobei das relative Verstellen das Volumen der Saugkammer (20) ändert,
wenigstens einen Pumpdurchlass (28), der sich von der Vakuumkammer (14), an einer Position zwischen den Enden dieser, zu der Saugkammer (20) erstreckt,
wenigstens einen Auslassdurchlass (29), der sich von der Saugkammer (20) erstreckt,
die erste Einwegventileinrichtung (32), die der Flüssigkeit ermöglicht, aus der Vakuumkammer (14) durch den oder durch jeden Pumpdurchlass (28) zu der Saugkammer (20) zu strömen, und
die zweite Einwegventileinrichtung, die der Flüssigkeit ermöglicht, aus der Saugkammer (20) durch den oder durch jeden Auslassdurchlass (29) an die Atmosphäre abgelassen zu werden.

2. Eine Vorrichtung (10, 100) nach Anspruch 1, wobei der oder jeder Pumpdurchlass (28) so angeordnet ist und das Pumpelement (16) so eingerichtet ist, dass das Verstellen des Pumpelements (16) in Richtung auf das Mundstück (12.4) des Behälters (12) das Volumen der Saugkammer (20) erhöht und verursacht, dass Luft aus der Vakuumkammer (14) durch den oder jeden Pumpdurchlass (28) in die Saugkammer (20) strömt und das Verstellen des Pumpelements (16) in der Richtung weg von dem Mundstück (12.4) des Behälters (12) das Volumen der Saugkammer (20) verringert und verursacht, dass die Luft aus der Saugkammer (20) durch den Auslassdurchlass (29) an die Atmosphäre abgelassen wird.

3. Vorrichtung nach Anspruch 1 oder 2, wobei der Behälter (12) eine zylindrische Wand (13) aufweist und sich der oder jeder Pumpdurchlass (28) durch die Wand (13) erstreckt und eine Öffnung in die Saugkammer (20) hinein aufweist, wobei sich die erste Einwegventileinrichtung in Form von wenigstens einem elastisch verformbaren Band (32) um die zylindrische Wand (13) herum erstreckt, um die oder jede Öffnung abzudecken.

4. Vorrichtung nach einem der vorhergehenden Ansprüche, wobei die zweite Einwegventileinrichtung einen O-Ring (24) umfasst, der eingerichtet ist, um eine luftdichte Abdichtung zwischen dem Pumpelement (16) und dem Behälter (12) bereitzustellen, wenn diese relativ zueinander verstellt sind, um das Volumen der Saugkammer (20) zu erhöhen und um das Ablassen von Luft durch wenigstens den einen Auslassdurchlass (29) zu ermöglichen, wenn das Pumpelement (16) und der Behälter (12) relativ zueinander verstellt werden, um das Volumen der Saugkammer (20) zu verringern.

5. Vorrichtung nach einem der vorherigen Ansprüche, wobei die Vorrichtung eine Vakuumbegrenzungseinrichtung (112) zum Begrenzen des Vakuums, das in der Vakuumkammer (14) gezogen werden kann, enthält.

6. Vorrichtung nach Anspruch 5, wobei die Vakuumbegrenzungseinrichtung ein in der Saugkammer (20) angeordnetes Hubbegrenzungselement (114), um den Hub, durch den das Pumpelement (16) relativ zu dem Behälter (12) verstellbar ist, zu begrenzen, enthält.

7. Vorrichtung nach einem der vorhergehenden Ansprüche, wobei das Pumpelement (16) an dem äußersten Ende seines Hubs nicht über die Enden des Behälters (12) hinaus hervorsteht.

8. Vorrichtung nach einem der vorhergehenden Ansprüche, wobei die Vorrichtung Dichtungseinrichtungen enthält, die in der Nähe des Mundstücks (12.4) angeordnet sind und die eingerichtet sind, um abdichtend an der Außenfläche eines in die Vakuumkammer (14) eingeführten menschlichen Penis anzuliegen.

9. Vorrichtung nach Anspruch 8, wobei die Dichtungseinrichtung einen Dichtungsverengungsring (34) umfasst, der in der Nähe des Mundstücks (12.4) aufsetzbar auf dem Behälter (12) befestigt ist, wobei der Verengungsring (34) einen im Allgemeinen ringförmigen Körper mit einer sich umlaufend erstreckenden Eingriffsanordnung (34.3) zum lösbaren abdichtenden Anliegen an einem Behälter (12) in der Nähe des Mundstücks (12. 4) und eine Verengungsringöffnung (34.1), die eingerichtet ist, um abdichtend an der Außenfläche eines menschlichen Penis anzuliegen, aufweist.

10. Vorrichtung nach Anspruch 9, wobei der Körper durch ein einheitliches Formen eines elastomeren Materials gebildet wird.

11. Vorrichtung nach Anspruch 9 oder 10, wobei die Verengungsringöffnung (34.1) einen Durchmesser zwischen 12 mm und 24 mm hat.
